# EUROPÄISCHE PATENTANMELDUNG

(11) **EP 4 230 134 A1**
(43) Veröffentlichungstag der Anmeldung: **23.08.2023**
(21) Anmeldenummer: 23156301.6
(22) Anmeldetag: 13.02.2023
(51) Int. Cl.: A61B 5/15

(54) **SCHUTZVORRICHTUNG FÜR DIE HAND EINES MEDIZINISCHEN PERSONALS BEI DER PUNKTION EINER NABELSCHNUR VON NEUGEBORENEN**

(30) Priorität: 16.02.2022 IT 202200002870
(71) Anmelder: PFM Medical AG, 50996 Köln (DE)
(72) Erfinder: Welfers, Pia, 41844 Wegberg (DE); Haase, Bianca, 72070 Tübingen (DE); Kox, Georg, 41844 Wegberg (DE); Rauch, Daniel, 45147 Essen (DE)
(74) Vertreter: Hohendorf Kierdorf Patentanwälte PartGmbB

(57) **Zusammenfassung**

Die Erfindung betrifft eine Schutzvorrichtung (1) für die Hand (2) eines medizinischen Personals bei der Punktion einer Nabelschnur (3) von Neugeborenen, umfassend:
eine Nabelschnurrinne (4) zur Aufnahme der Nabelschnur (3) des Neugeborenen,
zwei am oberen Rand der Nabelschnurrinne (4) angeordnete Flügel (5) als Handschutz, wobei sich die Flügel (5) jeweils seitlich von der Nabelschnurrinne (4) weg erstrecken, und
zwei Griffleisten (6), wobei die Griffleisten (6) an den beiden Flügeln (5) angeordnet sind und sich von den seitlichen Flügeln (5) jeweils eine Griffleiste (6) in der Tiefenrichtung der Nabelschnurrinne (4) erstreckt, wobei die Höhe der jeweiligen Griffleisten (6) wenigstens der Tiefe der Nabelschnurrinne (4) entspricht, so dass die Nabelschnurrinne (4) zwischen den beiden Griffleisten (6) angeordnet ist.

## Beschreibung

Die Erfindung betrifft eine Schutzvorrichtung für die Hand eines medizinischen Personals bei der Punktion einer Nabelschnur von Neugeborenen.

Nach aktuell geltenden Vorschriften und Leitlinien ist es notwendig und üblich, vor allem aus forensischen Gründen, postpartal den pH Wert aus dem Nabelschnurblut zu bestimmen, um Aufschluss über die peripartale Versorgung des Neugeborenen zu erhalten. Je später das Nabelschnurblut abgenommen wird, desto verfälschter ist der Wert. Daher wird aktuell direkt nach der Geburt abgenabelt. Dies Problematik kann umgangen werden, indem das Nabelschnurblut zur pH Bestimmung an der pulsierenden Nabelschnur abgenommen wird. Studien haben gezeigt, dass die Blutgaswerte nach Auspulsieren der Nabelschnur auf Grund der Kreißlaufumstellung und des zunehmend persistierenden Blutfluss in den Nabelschnurgefäßen einen azidotischen (sauren), nicht mit der reellen Situation des Kindes übereinstimmenden Wert ergeben, der eine Azidose (Übersäuerung) vermuten lässt.

Aus Studien ist bekannt, dass Neugeborene nach verspätetem Abnabeln höhere Eisenspeicher, die in der Regel bis zum ersten Lebensjahr ausreichen, aufweisen. Zudem erhalten spätabgenabelte Neugeborene durch diese Maßnahmen mehr Stammzellen, was sich wiederum positiv auf das Immunsystem, die Blutbildung und die Entwicklung des Zentrales Nervensystem (ZNS) des Neugeborenen auswirkt. Es konnten höhere Hämoglobinwerte, bessere neurologische und feinmotorische Entwicklungen beobachtet werden.

Aufgrund der Geburtsumgebung und der zeitlichen Vorgaben stellt die Blutentnahme im Rahmen des Spätabnabelns das medizinische Personal vor eine große Herausforderung. Die Mutter und/oder das Kind können sich bewegen, die Nabelschnur und die Umgebung sind durch verschiedenste Flüssigkeiten wie beispielsweise Fruchtwasser oder Blut glitschig und schwer handzuhaben. Es besteht daher die Gefahr, dass sich a) das medizinische Personal bei der Probenentnahme mit der Spritze selbst verletzt, oder b) Mutter und Kind verletzt werden könnten. Solche Verletzungen bedeuten gleichzeitig ein erhöhtes Infektionsrisiko. Diesbezüglich sei auch auf die EU-Richtlinie 2010/32/EU zur Vermeidung von Stich- und Schnittverletzungen im Gesundheitssektor und die Technischen Regeln für Biologische Arbeitsstoffe (TRBA) 250 verwiesen.

Um die Entnahme von Blutproben aus der Nabelschnur für das medizinische Personal sicherer zu machen, sind aus dem Stand der Technik unterschiedliche Vorrichtungen bekannt. So offenbart beispielsweise die WO 01/76660 A2 eine Schutzvorrichtung zur Aufnahme der Nabelschnur während der Punktion der Nabelschnur. Die Vorrichtung ist rinnenförmig und schützt insbesondere vor Verletzungen bei einem unbeabsichtigten Durchstechen der Nabelschnur. Die Nabelschnur wird in die rinnenförmige Schutzvorrichtung gelegt und vom medizinischen Personal mit einem Finger, insbesondere einem Daumen, fixiert. Stichverletzungen durch unerwartete Bewegungen von Mutter und/oder Kind werden jedoch nicht zuverlässig verhindert, insbesondere da der Finger zur Fixierung in unmittelbarer Nähe zur Punktionsstelle angeordnet ist.

Die FR 2 609 635 offenbart ebenfalls eine rinnenförmige Schutzvorrichtung, jedoch wird hierbei die Nabelschnur am distalen Ende und proximalen Ende mittels Klemmen an der Schutzvorrichtung fixiert. Die Nabelschnur muss also bei der Punktion nicht zusätzlich mit einem Finger fixiert werden. Jedoch muss die Nabelschnur mittels der Klemmen erst fixiert werden, bevor diese punktiert werden kann. Ferner wird durch die Klemmen der Blutfluss durch die Nabelschnur zumindest teilweise behindert, was zu verfälschten Blutergebnissen führen kann. Vergleichbare Schutzvorrichtungen sind aus der US 5,690,646 A und US 5,575,795 A bekannt.

Die FR 2 954 085 offenbart eine Vorrichtung zur Entnahme von Plazentablut nachdem die Nabelschnur vom Neugeborenen getrennt wurde. Eine Probenentnahme unmittelbar nach der Geburt ist gemäß der FR 2 954 085 nicht vorgesehen. Ferner umfasst die offenbarte Vorrichtung Klemmen zum Abklemmen der Nabelschnur, was zu verfälschten Blutergebnissen führen kann.

Die WO 2014/143664 A1 offenbart eine Klemme, welche auf die Nabelschnur geklemmt wird. Die Klemme umfasst eine Führung für die Punktionsnadel. Nachteilig an dieser Vorrichtung ist, dass die Punktionsnadel in die Führung eingeführt werden muss. Ferner verdeckt die Klemme die Einstichstelle und es ist für das medizinische Personal schwer zu überprüfen, ob die Punktionsnadel bereits in das Blutgefäß der Nabelschnur eingeführt wurde oder diese auch durchstochen wurde.

Ferner offenbart die EP 2 913 004 A1 eine rinnenförmige Schutzvorrichtung mit einem Befestigungsabschnitt und einem Punktionsabschnitt. Im Befestigungsabschnitt ist an der Unterseite der rinnenförmigen Schutzvorrichtung ein Griffteil angeordnet, um die Vorrichtung zwischen zwei Fingern einer Hand zu fixieren. Mit einem anderen Finger, vorzugsweise dem Daumen, wird die Nabelschnur auf der Oberseite der rinnenförmigen Schutzvorrichtung fixiert. Benachbart zu dem Befestigungsabschnitt ist der Punktionsabschnitt angeordnet. Ein Finger des medizinischen Personals befindet sich somit in unmittelbarer Nähe zur Punktionsstelle. Auch werden Stichverletzungen durch Bewegungen von Mutter und/oder Neugeborenem nicht sicher vermieden.

Der Erfindung liegt daher die Aufgabe zugrunde, das medizinische Personal ebenso wie Mutter und Kind bei der Punktion der Nabelschnur eines noch über die Nabelschnur mit der Mutter verbundenen Neugeborenen vor Stichverletzungen zu schützen, insbesondere bei unvorhersehbaren Bewegungen von Mutter und/oder Kind, ohne dass die Blutergebnisse beeinflusst werden.

Die Aufgabe wird erfindungsgemäß gelöst durch eine Schutzvorrichtung für die Hand eines medizinischen Personals bei der Punktion einer Nabelschnur von Neugeborenen, umfassend:
eine Nabelschnurrinne zur Aufnahme der Nabelschnur des Neugeborenen,
zwei am oberen Rand der Nabelschnurrinne angeordnete Flügel als Handschutz, wobei sich die Flügel jeweils seitlich von der Nabelschnurrinne weg erstrecken, und
zwei Griffleisten, wobei die Griffleisten an den beiden Flügeln angeordnet sind und sich von den seitlichen Flügeln jeweils eine Griffleiste in der Tiefenrichtung der Nabelschnurrinne erstreckt, wobei die Höhe der jeweiligen Griffleisten wenigstens der Tiefe der Nabelschnurrinne entspricht, so dass die Nabelschnurrinne zwischen den beiden Griffleisten angeordnet ist.

Die erfindungsgemäße Schutzvorrichtung kann vom medizinischen Personal mittels der beiden Griffleisten in einer Hand gehalten werden. Die Nabelschnurrinne, die zwischen den beiden Griffleisten angeordnet ist, verläuft dabei durch die Handinnenfläche des medizinischen Personals, was für die Punktion der Nabelschnur einer besonders ergonomischen Haltung entspricht. Die Nabelschnurrinne verhindert Stichverletzungen durch ein unbeabsichtigtes Durchstechen der Nabelschnur. Die am oberen Rand der Nabelschnurrinne angeordneten Flügel schützen die Hand des medizinischen Personals vor Stichverletzungen durch ein Abrutschen der Nadel oder anderen ungewollte Bewegungen, beispielsweise verursacht durch Bewegungen der Mutter oder des Neugeborenen. Die Haltehand des medizinischen Personals befindet sich somit unterhalb der Nabelschnurrinne und wird von oben durch die seitlichen Flügel geschützt. Aufgrund dieser Anordnung kann die Nabelschnur an jeder Stelle der Nabelschnurrinne punktiert werden, insbesondere an einer Stelle, wo ein entsprechendes Gefäß der Nabelschnur zur Blutentnahme gut sichtbar ist. Im Gegensatz kann beispielsweise beim Stand der Technik gemäß der EP 2 913 004 A1 die Nabelschnur im Punktionsbereich der Nabelschnurrinne punktiert werden, welcher sich ferner in unmittelbarer Nähe zum Befestigungsabschnitt befindet, wo das medizinische Personal die Nabelschnur auf der Oberseite der Nabelschnurrinne mit einem Finger fixiert.

Nach einer bevorzugten Variante der Erfindung erstrecken sich die zwei Flügel über die gesamte Länge der Nabelschnurrinne. Dadurch wird die Haltehand des medizinischen Personals vor Stichverletzungen geschützt, unabhängig davon, an welcher Stelle der Nabelschnurrinne die Nabelschnur punktiert wird.

Gemäß einer vorteilhaften erfindungsgemäßen Variante erstrecken sich die zwei Griffleisten über die gesamte Länge der Nabelschnurrinne. Derartige Griffleisten ermöglichen eine besonders gute Handhabung der erfindungsgemäßen Schutzvorrichtung, insbesondere einen sicheren Halt in der Hand des medizinischen Personals. Das medizinische Personal kann die erfindungsgemäße Schutzvorrichtung somit entlang der gesamten Länge greifen, unabhängig von der Größe der Hand des medizinischen Personals, da jeder die erfindungsgemäße Schutzvorrichtung dort greifen kann, wo es für ihn angenehm ist. Ferner kann die erfindungsgemäße Schutzvorrichtung stabil auf den beiden Griffleisten abgelegt werden.

Die zwei Griffleisten haben den zusätzlichen Vorteil, dass die erfindungsgemäße Vorrichtung gleichermaßen von Rechts- und Linkshändern benutzt werden kann.

In einer zweckmäßigen Variante der Erfindung weisen die Griffleisten jeweils wenigstens eine seitliche Einbuchtung auf, für einen Finger der Hand des medizinischen Personals. Durch die Einbuchtungen kann die erfindungsgemäße Schutzvorrichtung noch sicherer in der Hand des medizinischen Personals gehalten werden. Insbesondere sind die Einbuchtungen im proximalen Drittel der Schutzvorrichtung angeordnet, beispielsweise zur Aufnahme des Daumens und Zeigefingers der Hand des medizinischen Personals. Proximal im Sinne der Erfindung ist das der Mutter zugewandte Ende der Schutzvorrichtung. Distal im Sinne der Erfindung ist das dem medizinischen Personal zugewandte Ende der Schutzvorrichtung.

Nach einer besonders vorteilhaften Variante ist die Nabelschnurrinne zum proximalen Ende in der Tiefenrichtung der Nabelschnurrinne abgewinkelt. Insbesondere ist die Abwinkelung abgerundet und nicht eckig. Durch die Abwinkelung wird die Nabelschnur besser in der Nabelschnurrinne gehalten und die Nabelschnur wird an den Enden der Schutzvorrichtung nicht abgeknickt, sondern im Bereich des üblichen Verlaufs gestützt.

Zweckmäßigerweise liegt der Winkel des proximalen Endes zum distalen Ende der Nabelschnurrinne zwischen 30 Grad und 60 Grad, insbesondere zwischen 40 Grad und 50 Grad.

Gemäß einer Variante der Erfindung weisen die seitlichen Flügel am distalen Ende die größte seitliche Erstreckung auf und werden zum proximalen Ende kontinuierlich schmaler. Am distalen Ende ist beim bestimmungsgemäßen Gebrauch der erfindungsgemäßen Vorrichtung die Handfläche des medizinischen Personals angeordnet, während am proximalen Ende die Fingerspitzen angeordnet sind. Da die Fingerspitzen insgesamt schmaler sind als die Handfläche und auch übereinander gehalten werden können, können die seitlichen Flügel am proximalen Ende weniger breit ausgebildet sein als am distalen Ende und dennoch denselben Schutz bereitstellen. Eine zum proximalen Ende konisch zulaufende Schutzvorrichtung, also eine zum proximalen Ende schmaler werdende Schutzvorrichtung, ist weniger sperrig und ergonomischer in der Handhabung. Des Weiteren kann die Schutzvorrichtung so besser in der Leiste der Mutter abgelegt werden.

In einer erfindungsgemäßen Variante weist die Nabelschnurrinne am distalen Ende und/oder am proximalen Ende eine Verengung zur Fixierung der Nabelschnur auf. Insbesondere ist die Verengung am distalen Ende und/oder proximalen Ende v-förmig ausgebildet. Eine derartige Verengung hilft die Nabelschnur durch ihr Eigengewicht in der Nabelschnurrinne zu fixieren, ohne dass die Nabelschnur abgeklemmt bzw. die Durchblutung der Nabelschnur vermindert wird.

Nach einer bevorzugten Variante der Erfindung ist die Innenseite der Nabelschnurrinne weicher ausgebildet als eine Punktionsnadel, so dass die Punktionsnadel beim Durchstechen der Nabelschnur in der Innenseite der Nabelschnurrinne fixiert werden kann. Insbesondere ist die Innenseite der Nabelschnurrinne zu diesem Zweck flexibel ausgebildet. Dies hat den Vorteil, dass nach der Punktion und Probenentnahme die Punktionsnadel durch die Nabelschnur gestochen und in der Innenseite der Nabelschnurrinne fixiert werden kann. Dort kann die Punktionsnadel verbleiben, bis die Nabelschnur getrennt und beiseitegeschafft wurde. Es werden somit auch Stichverletzungen nach der Probenentnahme sicher verhindert. Beispielsweise weist die Innenseite der Nabelschnurrinne eine extra Beschichtung auf oder die Nabelschnurrinne ist aus einem weicheren Material, insbesondere Kunststoff, hergestellt, weist aber eine ausreichende Wandstärke auf, um ein Durchstechen mit der Punktionsnadel zu verhindern.

Zudem werden in der Regel mehrere Blutproben aus der Nabelschnur entnommen. Beispielsweise wird zunächst die Nabelarterie punktiert, um den arteriellen pH-Wert zu bestimmen. Nachfolgend kann die Nabelvene punktiert werden, um den venösen pH-Wert zu bestimmen. Zusätzlich kann noch Blut für ein EDTA Röhrchen zur Blutgruppenbestimmung oder ein Serum für Serologien aus der Nabelschnur entnommen werden. Die dabei verwendeten Punktionsnadel können in der vorteilhaften Variante in der weicheren Innenseite der Nabelschnurrinne fixiert werden.

Eine erfindungsgemäße Schutzvorrichtung für die Hand eines medizinischen Personals bei der Punktion einer Nabelschnur von Neugeborenen hat beispielsweise eine Länge zwischen 10 cm und 20 cm, insbesondere ungefähr 15 cm. Die Nabelschnurrinne der erfindungsgemäßen Schutzvorrichtung ist dabei gewölbt, U-förmig, polygonal oder dergleichen. Vorzugsweise hat die Nabelschnurrinne eine obere Breite von 15 mm bis 25 mm, insbesondere ungefähr 20 mm. Die seitlichen Flügel haben eine Breite von 1,0 cm bis 5,0 cm, vorzugweise ungefähr 2,5 cm. Insbesondere weisen die seitlichen Flügel am distalen Ende die größte seitliche Erstreckung auf und werden zum proximalen Ende kontinuierlich schmaler. Beispielsweise weisen die seitlichen Flügel am distalen Ende eine Breite von 2,5 cm bis 5,0 cm auf und verjüngen sich zum proximalen Ende auf 1,0 cm bis 2,5 cm.

Nachfolgend wird die Erfindung anhand von in den Figuren dargestellten Ausführungsbeispielen näher erläutert. Es zeigen:
- Fig. 1: eine perspektivische Draufsicht auf eine erfindungsgemäße Schutzvorrichtung für die Hand eines medizinischen Personals bei der Punktion einer Nabelschnur von Neugeborenen,
- Fig. 2: eine perspektivische Ansicht von unten auf die erfindungsgemäße Schutzvorrichtung aus Fig. 1,
- Fig. 3: eine Seitenansicht der erfindungsgemäßen Schutzvorrichtung aus den Figuren 1 und 2, und
- Fig. 4: eine erfindungsgemäße Schutzvorrichtung für die Hand eines medizinischen Personals bei der Punktion einer Nabelschnur eines Neugeborenen.

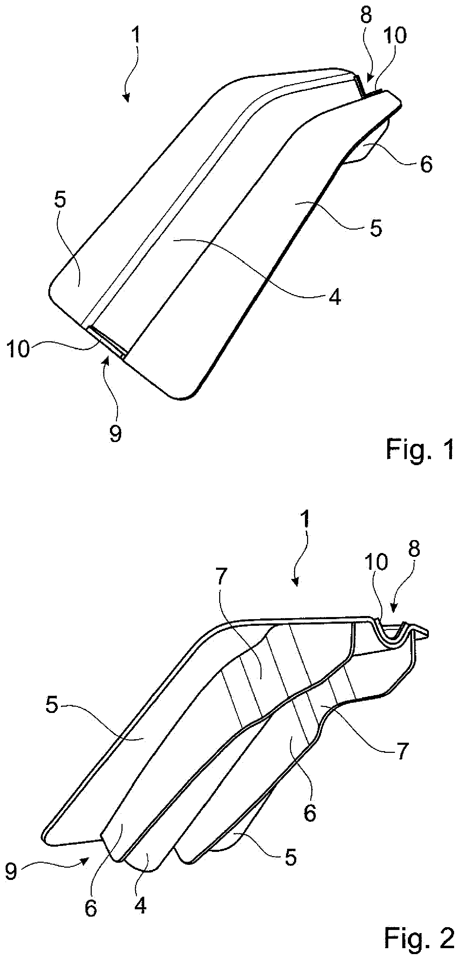

Fig. 1 zeigt eine perspektivische Draufsicht auf eine erfindungsgemäße Schutzvorrichtung 1 für die Hand 2 eines medizinischen Personals bei der Punktion einer Nabelschnur 3 von Neugeborenen. Die Schutzvorrichtung 1 umfasst eine Nabelschnurrinne 4 zur Aufnahme der Nabelschnur des Neugeborenen und zwei am oberen Rand der Nabelschnurrinne 4 angeordnete Flügel 5 als Handschutz. Die Flügel 5 erstrecken sich in der Gebrauchslage der Schutzvorrichtung 1 seitlich von der Nabelschnurrinne 4 weg.

Die Schutzvorrichtung 1 umfasst ferner zwei an den beiden Flügeln 5 angeordnete Griffleisten 6. Von den seitlichen Flügeln 5 erstreckt sich jeweils eine Griffleiste 6 in der Tiefenrichtung der Nabelschnurrinne 4. Die Höhe der beiden Griffleisten 6 entspricht wenigstens der Tiefe der Nabelschnurrinne 4. Die Nabelschnurrinne 4 ist somit zwischen den beiden Griffleisten 6 angeordnet.

Die erfindungsgemäße Schutzvorrichtung 1 weist beispielsweise eine Länge zwischen 10 cm und 20 cm auf, insbesondere ungefähr 15 cm. Die Nabelschnurrinne 4 der Schutzvorrichtung 1 ist beispielsweise gewölbt, U-förmig oder polygonal und hat zweckmäßigerweise eine obere Breite von 15 mm bis 25 mm, insbesondere ungefähr 20 mm. Die Nabelschnurrinne weist gemäß dem in Fig. 1 dargestellten Ausführungsbeispiel am distalen Ende 9 und am proximalen Ende 8 jeweils eine Verengung 10 zur Fixierung der Nabelschnur 3 auf. Beispielsweise ist diese Verengung 10 V-förmig ausgebildet. Die Verengung 10 sorgt für eine bessere Fixierung der Nabelschnur 3 in der Nabelschnurrinne 4, wobei die Verengung 10 jedoch so ausgebildet ist, dass die Nabelschnur 3 und insbesondere der Blutfluss durch die Nabelschnur 3 nicht abgeklemmt wird.

Wie der Fig. 1 entnommen werden kann, erstrecken sich die zwei Flügel 5 über die gesamte Länge der Nabelschnurrinne 4. Die zwei Flügel 5 weisen am distalen Ende 9 der Schutzvorrichtung 1 eine größere seitliche Erstreckung auf als am proximalen Ende 8. Insbesondere werden die seitlichen Flügel 5 zum proximalen Ende 8 kontinuierlich schmaler. Beispielsweise weisen die zwei Flügel 5 am distalen Ende 9 eine Breite von 2,5 cm bis 5,0 cm auf und am proximalen Ende eine Breite von 1,0 cm bis 2,5 cm.

Nach einer bevorzugten Variante der Erfindung ist die Innenseite der Nabelschnurrinne 4 weicher ausgebildet als eine Punktionsnadel 11, so dass die Punktionsnadel 11 beim Durchstechen der Nabelschnur 3 in der Innenseite der Nabelschnurrinne 4 fixiert und gleichzeitig der Austritt von Nabelschnurblut reduziert werden kann. Insbesondere ist die Innenseite der Nabelschnurrinne 4 zu diesem Zweck flexibel ausgebildet. Dies hat den Vorteil, dass nach der Punktion und Probenentnahme die Punktionsnadel 11 durch die Nabelschnur 3 gestochen und in der Innenseite der Nabelschnurrinne 4 fixiert werden kann. Dort kann die Punktionsnadel 11 verbleiben, bis die Nabelschnur 3 getrennt und beiseitegeschafft wurde. Es werden somit auch Stichverletzungen nach der Probenentnahme sicher verhindert. Beispielsweise weist die Innenseite der Nabelschnurrinne 4 eine extra Beschichtung auf oder die Nabelschnurrinne 4 ist aus einem weicheren Material, insbesondere Kunststoff, hergestellt, weist aber eine ausreichende Wandstärke auf, um ein Durchstechen mit der Punktionsnadel 11 zu verhindern.

Fig. 2 zeigt eine perspektivische Ansicht von unten auf die erfindungsgemäße Schutzvorrichtung 1 aus Fig. 1. Wie insbesondere der Fig. 2 entnommen werden kann, erstrecken sich die zwei Griffleisten 6 nahezu über die gesamte Länge der Nabelschnurrinne 4 bzw. der Schutzvorrichtung 1. Die beiden Griffleisten 6 weisen jeweils eine Einbuchtung 7 auf, für jeweils einen Finger der Hand 2 des medizinischen Personals, insbesondere dem Daumen und Zeigefinger der Hand 2. Die Einbuchtungen 7 sind ungefähr im proximalen Drittel der Schutzvorrichtung 1 angeordnet.

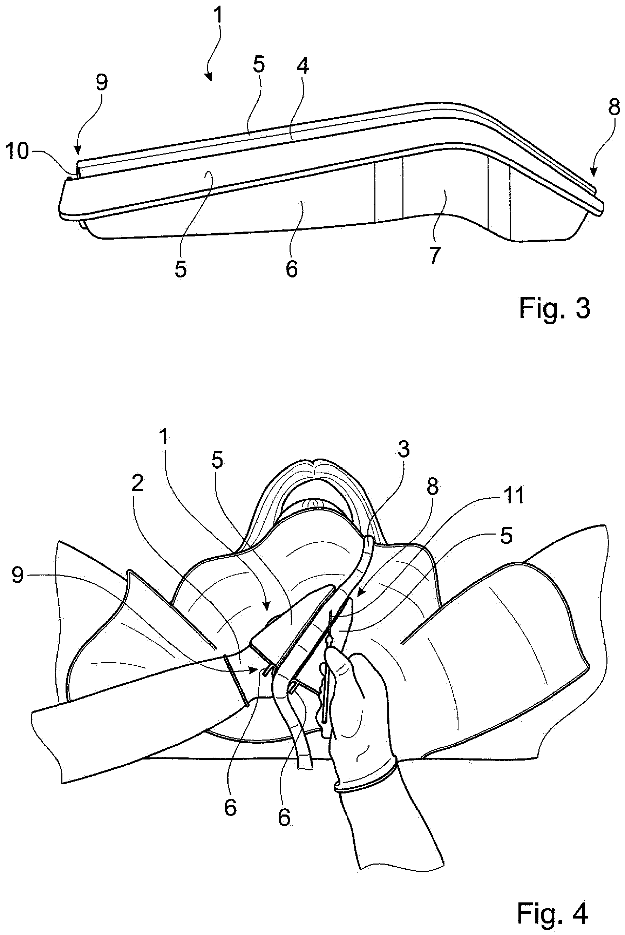

Fig. 3 zeigt eine Seitenansicht der erfindungsgemäßen Schutzvorrichtung 1 aus den Figuren 1 und 2. Der Fig. 3 ist besonders gut zu entnehmen, dass die Nabelschnurrinne 4 zum proximalen Ende 8 in der Tiefenrichtung der Nabelschnurrinne 4 abgewinkelt ist, also in der Gebrauchslage der Schutzvorrichtung 1 nach unten. Der Winkel des proximalen Endes 8 zum distalen Ende 9 der Nabelschnurrinne 4 liegt erfindungsgemäß zwischen 30 Grad und 60 Grad, insbesondere zwischen 40 Grad und 50 Grad.

Der Fig. 3 lässt sich ferner entnehmen, dass die Einbuchtungen 7 an den Griffleisten 6 ungefähr im Bereich der Abwinkelung angeordnet sind und dass die erfindungsgemäße Schutzvorrichtung 1 stabil auf den beiden Griffleisten 6 abgelegt werden kann. Da die beiden Griffleisten auf gegenüberliegenden Seiten der Nabelschnurrinne 4 angeordnet sind, kann die Nabelschnur 3 auch im abgelegten Zustand punktiert werden, insbesondere ohne, dass die Schutzvorrichtung 1 seitlich wegkippt.

Fig. 4 zeigt eine erfindungsgemäße Schutzvorrichtung 1 für die Hand 2 eines medizinischen Personals bei der Punktion einer Nabelschnur 3 eines Neugeborenen. Das medizinische Personal kann die erfindungsgemäße Schutzvorrichtung 1 mittels der in Gebrauchslage unterhalb der beiden Flügel 5 angeordneten Griffleisten 6 in einer Hand 2 halten. Die Nabelschnur 3 des Neugeborenen wird in der Nabelschnurrinne 4 platziert und kann mit der anderen Hand des medizinischen Personals mittels einer Punktionsnadel 11 punktiert werden. Die Nabelschnurrinne 4 schützt die Hand 2 des medizinischen Personals vor Stichverletzungen, verursacht durch ein Durchstechen der Nabelschnur 3. Die seitlichen Flügel 5 schützen die Hand 2 des medizinischen Personals vor Stichverletzungen, verursacht durch ein Abrutschen oder ähnliches von der Nabelschnurrinne 4, beispielsweise aufgrund von unvorhergesehenen Bewegungen der Mutter und/oder des Neugeborenen. Da die Hand 2 zum Halten der erfindungsgemäßen Schutzvorrichtung 1 komplett unterhalb der Flügel 5 angeordnet ist, kann die in der Nabelschnurrinne 4 platzierte Nabelschnur 3 irgendwo entlang der kompletten Länge der Nabelschnurrinne 4 punktiert werden, ohne das Risiko von Stichverletzungen.

### Bezugszeichenliste

- 1: Schutzvorrichtung
- 2: Hand
- 3: Nabelschnur
- 4: Nabelschnurrinne
- 5: Flügel (Handschutz)
- 6: Griffleiste
- 7: Einbuchtung der Griffleiste
- 8: proximales Ende
- 9: distales Ende
- 10: Verengung
- 11: Punktionsnadel

## Patentansprüche

1. Schutzvorrichtung (1) für die Hand (2) eines medizinischen Personals bei der Punktion einer Nabelschnur (3) von Neugeborenen, umfassend:
eine Nabelschnurrinne (4) zur Aufnahme der Nabelschnur (3) des Neugeborenen,
zwei am oberen Rand der Nabelschnurrinne (4) angeordnete Flügel (5) als Handschutz, wobei sich die Flügel (5) jeweils seitlich von der Nabelschnurrinne (4) weg erstrecken, und
zwei Griffleisten (6), wobei die Griffleisten (6) an den beiden Flügeln (5) angeordnet sind und sich von den seitlichen Flügeln (5) jeweils eine Griffleiste (6) in der Tiefenrichtung der Nabelschnurrinne (4) erstreckt, wobei die Höhe der jeweiligen Griffleisten (6) wenigstens der Tiefe der Nabelschnurrinne (4) entspricht, so dass die Nabelschnurrinne (4) zwischen den beiden Griffleisten (6) angeordnet ist.

2. Schutzvorrichtung (1) nach Anspruch 1,
wobei sich die zwei Flügel (5) über die gesamte Länge der Nabelschnurrinne (4) erstrecken.

3. Schutzvorrichtung (1) nach Anspruch 1 oder Anspruch 2,
wobei sich die zwei Griffleisten (6) über die gesamte Länge der Nabelschnurrinne (4) erstrecken.

4. Schutzvorrichtung (1) nach einem der Ansprüche 1 bis 3,
wobei die Griffleisten (6) jeweils wenigstens eine seitliche Einbuchtung (7) aufweisen, für einen Finger der Hand (2) des medizinischen Personals.

5. Schutzvorrichtung (1) nach einem der Ansprüche 1 bis 4,
wobei die Nabelschnurrinne (4) zum proximalen Ende (8) in der Tiefenrichtung der Nabelschnurrinne (4) abgewinkelt ist.

6. Schutzvorrichtung (1) nach Anspruch 5,
wobei der Winkel des proximalen Endes (8) zum distalen Ende (9) der Nabelschnurrinne (4) zwischen 30 Grad und 60 Grad liegt, insbesondere zwischen 40 Grad und 50 Grad.

7. Schutzvorrichtung (1) nach einem der Ansprüche 1 bis 6,
wobei die seitlichen Flügel (5) am distalen Ende (9) die größte seitliche Erstreckung aufweisen und zum proximalen Ende (8) kontinuierlich schmaler werden.

8. Schutzvorrichtung (1) nach einem der Ansprüche 1 bis 7,
wobei die Nabelschnurrinne (4) am distalen Ende (9) und/oder am proximalen Ende (8) eine Verengung (10) zur Fixierung der Nabelschnur (3) aufweist.

9. Schutzvorrichtung (1) nach Anspruch 8,
wobei die Verengung (10) am distalen Ende (9) und/oder proximalen Ende (8) V-förmig ausgebildet ist.

10. Schutzvorrichtung (1) nach einem der Ansprüche 1 bis 9,
wobei die Innenseite der Nabelschnurrinne (4) weicher ausgebildet ist als eine Punktionsnadel (11), so dass die Punktionsnadel (11) beim Durchstechen der Nabelschnur (3) in der Innenseite der Nabelschnurrinne (4) fixiert werden kann.
